(19) Europäisches Patentamt
European Patent Office
Office européen des brevets

(11) Publication number: 0 143 627
A1

# (12) EUROPEAN PATENT APPLICATION

(21) Application number: 84308161.3

(22) Date of filing: 26.11.84

(51) Int. Cl.⁴: C 07 C 143/72, C 07 C 143/70, C 07 C 145/00, C 07 D 239/38

(30) Priority: 28.11.83 US 555765

(43) Date of publication of application: 05.06.85 Bulletin 85/23

(84) Designated Contracting States: BE CH DE FR GB IT LI LU NL SE

(71) Applicant: ELI LILLY AND COMPANY, 307, East McCarty Street, Indianapolis Indiana 46285 (US)

(72) Inventor: Bingham, Alpheus, Jr., 1820 Arcadia Drive, Lafayette Indiana 47905 (US)
Inventor: Pirkle, William Howard, 501 N. Russell Street, Champaign Illinois 61821 (US)

(74) Representative: Tapping, Kenneth George et al, Lilly Industries Limited Patent Department Erl Wood Manor, Windlesham Surrey, GU20 6PH (GB)

## (54) An improved process for preparing sulfonamides.

(57) The present invention relates to a convenient and economical process for preparing sulfonamide derivatives in which an organometallic compound is reacted, in sequence, with sulfur dioxide, chlorine or bromine and ammonia.

# AN IMPROVED PROCESS FOR PREPARING SULFONAMIDES

One skilled in the art frequently deals with highly reactive, volatile and corrosive reagents such as the sulfonyl halides and the halogens such as chlorine and bromine. To alleviate the potential hazards involved in working with reagents of these types, it is desirable to avoid as much human manipulation as possible. In particular, one pot, in situ generation and utilization of intermediate products is preferred.

Further, for commercial large scale production those skilled in the art continually search for techniques involving minimal manipulation using cheap, readily available starting materials.

The present invention, therefore provides a convenient one-pot synthesis of a sulfonamide employing readily available starting materials and advantageous synthetic techniques suitable for industrial scale preparations.

In particular, this invention provides an economical and convenient process for preparing a sulfonamide of Formula (1)

$$R^1\text{-}SO_2\text{-}NH_2 \qquad (1)$$

in which $R^1$ is $C_1$-$C_{10}$ alkyl, phenyl, substituted phenyl or a 6-membered heteroaromatic ring containing one or two nitrogen atoms and the remainder carbon atoms, such process comprising:

(a) contacting an organometallic compound of the formula

$$R^1\text{-}M$$

in which M is an alkali metal or $M^1X$, in which $M^1$ is Cd, Zn or Mg and X is halogen, with sulfur dioxide to give an organosulfinate metal salt;

(b) treating the organosulfinate metal salt of step (a) with chlorine or bromine to afford a sulfonyl halide derivative; and

(c) treating the sulfonyl halide with ammonia.

Further, there is provided a process for preparing a sulfonyl halide of Formula (2)

$$R^1\text{-}SO_2\text{-}X \qquad (2)$$

in which $R^1$ is $C_1$-$C_{10}$ alkyl, phenyl, substituted phenyl or a 6-membered heteroaromatic ring containing one or two nitrogen atoms and the remainder carbon atoms and X is chlorine or bromine which comprises treating an organosulfinate metal salt of the formula

$$R^1\text{-}SO_2\text{-}M$$

in which M is an alkali metal or $M^1X$, in which $M^1$ is Cd, Zn or Mg and X is halogen, with chlorine or bromine.

The term $C_1$-$C_{10}$ alkyl, as used herein, represents a straight or branched alkyl chain having from one to ten carbon atoms. Typical $C_1$-$C_{10}$ alkyl groups include groups such as methyl, ethyl, n-propyl, isopropyl, n-butyl, sec-butyl, t-butyl, n-pentyl, sec-hexyl, n-heptyl, isooctyl, n-nonyl, or n-decyl.

"Substituted phenyl" refers to a phenyl ring bearing one, two or three substituents which are inert to the reaction conditions used in the process. Suitable substituents may include, for example, $C_1$-$C_4$ alkyl, $C_1$-$C_4$ alkoxy, an ethylene ketal substituent, nitro, or amino.

The term 6-membered heteroaromatic ring containing one or two nitrogen atoms and the remainder carbon atoms includes pyridine, pyrazine, pyrimidine and pyridazine.

The first step of the present process comprises the sulfonation of an organometallic compound $R^1$-M as defined above with sulfur dioxide in a suitable solvent under dry conditions to provide an organosulfinate metal salt. The organometallic compound employed may possess a metal halide substituent but will preferably possess an alkali metal substitutent. When an organcmetallic halide is employed typical metals are zinc, cadmium and especially magnesium. Suitable halogen substituents in the organometallic halide include chlorine and especially bromine. Typical alkali metals suitable for use in the present process are lithium, sodium and potassium. Lithium is preferred.

Sulfur dioxide may be added to the reaction mixture as a gas if used at ambient temperature and pressure, or as a liquid if it is cooled to a temperature below approximately -10°C. At least one equivalent of sulfur dioxide must be added to the reaction mixture although typically an excess is employed, for example up to 10 equivalents or more. Generally the sulfur dioxide is added to the reaction mixture until no further reaction is apparent. Any excess sulfur dioxide must be removed before the subsequent addition of the halogenating agent. This usually is conducted by passing an inert gas such as nitrogen or argon through the reaction vessel for a short period of time.

The sulfonation of the organometallic starting material is carried out in a suitable solvent and under dry conditions. Suitable solvents may include ethers such as tetrahydrofuran and diethyl ether, as well as most hydrocarbon solvents. Typical hydrocarbon solvents may be the aromatic hydrocarbons, such as benzene, toluene and the xylenes, in addition to the aliphatic hydrocarbons, such as pentane, hexane, 2,3-dimethylbutane, or heptane. Of these, tetrahydrofuran is the solvent of choice. The solvent should be water free and the reaction vessel void of any residual water. This may be accomplished by heating the vessel to drive off any water and allowing it to cool in an anhydrous atmosphere. During the reaction, use of an inert atmosphere is preferred. Generally an inert gas such as nitrogen or argon is passed through the reaction vessel.

The sulfonation step is typically complete after about 1 to 30 minutes when conducted at a temperature in the range of from about -110°C to 40°C. The organosulfinate metal salt thus formed is used directly without isolation in the following halogenation procedure.

The second step of the process involves halogenating the organosulfinate metal salt with chlorine or bromine to give the sulfonyl halide derivative. Dry conditions should be maintained throughout this step of the process. Typically one equivalent of gaseous bromine, or especially chlorine, is bubbled below the surface of

the reaction mixture, although amounts in excess of one equivalent appear to do no harm. The reaction is conducted at a temperature in the range of from about -110°C to 100°C, more preferably from about -50°C to 25°C. While use of the same solvent in this step as employed in the prior sulfonation step is preferred, the halogenation reaction also may be conducted in a halogenated hydrocarbon solvent. Examples of such solvents may include dichloromethane, chloroform, or 1,2-dibromoethane.

The reaction is complete when no exothermic reaction is apparent upon the continued addition of the gaseous halogenating agent. As with the sulfonation step described above, excess reagent must be removed before the last step of the process, typically by passing an inert gas through the reaction vessel. The product thus formed is used in situ in the final step of the process of the invention.

The final step of the present process involves treating the obtained sulfonyl halide with ammonia to provide the desired sulfonamide derivative. For this step the inert atmosphere is not necessary and preferably is not employed. The ammonia may be gaseous or may be used in a liquid form such as aqueous ammonium hydroxide or ammonia in acetone. Typically at least one equivalent, and preferably an excess, of ammonia is employed. The reaction usually is complete after about 1 minute to 2 hours when conducted at a temperature in the range of from -10°C to about 100°C. The sulfonamide then may be isolated by either collecting the precipitated solid by filtration or evaporating the reaction

mixture to dryness. The product then may be purified further if desired by either crystallization from common solvents or purification over solid supports such as silica gel or alumina, or by other means known to those skilled in the art.

The compounds prepared by the process of the present invention are useful for a variety of purposes. For example, U.S. Patent No. 3,320,312 discloses various phenylsulfonyl cycloalkylureas having hypoglycemic properties prepared from the corresponding benzenesulfonamide and isocyanate analogs. Certain of the sulfonamide derivatives prepared by the present process can be converted also to the corresponding sulfonyl isocyanate and reacted with an appropriate amine to prepare compounds having an agrichemical utility. The sulfonyl isocyanate generally is prepared by reacting the sulfonamide with phosgene in the presence of n-butyl isocyanate at reflux in a solvent such as chlorobenzene. See, e.g., U.S. Patent No. 4,169,719 directed to N-(heterocyclicaminocarbonyl)arylsulfonamides as herbicides. The present process may be used also to prepare dinitrosulfanilamide herbicides as exemplified in U.S. Patent No. 3,367,949.

The organometallic compounds employed as starting materials for the present process either are commercially available or readily prepared by known procedures. The starting materials having an alkali metal substituent are typically prepared by reacting an organohalide with an alkylmetal reagent according to the following scheme:

$$R^1X + R^2M \rightarrow R^1M + R^2X$$

in which $R^1$ is as defined above, M is an alkali metal, X is halogen and $R^2$ is $C_1$-$C_6$ alkyl.

Preferred halogen atoms are bromine and chlorine and the preferred alkali metal reagent is n-butyllithium. This reaction is conducted in a suitable solvent at low temperatures in the presence of an inert atmosphere. Suitable solvents should be water free and may include ethers such as diethyl ether or especially tetrahydrofuran. The inert atmosphere generally is obtained by passing either nitrogen or argon through the reaction vessel. Generally one equivalent of organohalide is combined with one or more equivalents of an alkyl metal reagent. The reaction substantially is complete after about 10 minutes to 24 hours when conducted at a temperature in the range of from about -100°C to 0°C. Preferably, the organometallic compound prepared is used directly in the present process without isolation.

The organometallic halide starting materials are prepared also by procedures well-known to those skilled in the art or by methods analogous to such prior art procedures. Generally, an organohalide is treated with a suitable metal according to the following scheme:

$$R^1X + M^1 \rightarrow R^1M^1X$$

in which $R^1$ and X are as defined above and $M^1$ is zinc, cadmium or especially magnesium.

Typically this reaction is conducted by combining about one equivalent of organohalide with from one equivalent to a slight excess of metal in a suitable solvent under inert dry conditions. Suitable solvents should be water free and may include the ethers such as diethyl ether and especially tetrahydrofuran. Inert atmosphere typically is provided by either nitrogen or argon. The reaction usually is initiated by grinding the metal in a suitable solvent in the reaction flask and adding a small amount of an initiating agent such as iodine or 1,2-dibromoethane. Generally the solution is allowed to reflux without the aid of external heat by the mere dropwise addition of the organohalide in a suitable solvent to the stirred reaction mixture. After addition of the organohalide is complete, the solution usually is refluxed for about 30 to 90 minutes by the application of an external heat source. The organometallic halide may be used directly without further purification in the first step of the process of the present invention.

The preparation of the cadmium reagents in which $M^1$ above is cadmium are usually prepared by transmetallation of a Grignard reagent with cadmium chloride according to well-known procedures.

The following non-limiting examples are provided to further illustrate the invention.

Example 1

Benzenesulfonamide

A 100 ml 3-neck round-bottom flask which had been dried at 100°C under vacuum and fitted with a condenser, thermometer and mechanical stirrer, was charged with 1.07 g (44.0 mmol) of magnesium turnings and 6 ml of tetrahydrofuran. A small amount of a solution of 6.58 g bromobenzene (99%, 41.5 mmol) and 34 ml tetrahydrofuran was added to the flask. The reaction was initiated by breaking a few of the magnesium turnings followed by the addition of a small iodine crystal and the application of heat. The remainder of the bromobenzene solution was added over 30 minutes while a minimal amount of additional heat was required to maintain reflux. Following the addition of the bromobenzene solution, the reaction mixture was heated at reflux for an additional 45 minutes. The solution was cooled to 0-5°C whereupon 2.1 ml sulfur dioxide (47.0 mmol) was added dropwise, from an addition funnel topped by a dry ice-filled cold finger condenser, to the Grignard mixture. The reaction mixture was cooled to about -5°C during which time a dynamic nitrogen purge was used. Chlorine gas was bubbled gently below the reaction mixture surface until the vigorous exothermic reaction subsided. The reaction mixture was cooled to about -9°C and the chlorine gas was removed. A solution of 5.36 g 28% ammonia in water

and 20 ml acetone was added dropwise to the reaction mixture over about 30 minutes. The precipitated solid was filtered and washed with acetone. The combined mother liquor and washes were concentrated under vacuum, slurried in water and acidified with 12 N HCl. The solid slurry was chilled, filtered, washed with water and hexane, and the resulting solid was dried in a vacuum oven to obtain 3.24 g of benzenesulfonamide. The proton NMR was identical to that of an authentic sample. mp = 150-152°C (uncorr.). The infrared spectrum also was consistent with the structure of benzenefulfonamide.

Example 2

5-Pyrimidinesulfonamide

A 100 ml three-neck round-bottom flask (air dried at 115°C) was charged with 5.13 g of 5-bromopyrimidine (99.2%, 32.0 mmol) and 60 ml of tetrahydrofuran (dried over molecular sieves). The solution was cooled to a temperature in the range of about -95° to -100°C whereupon 21.0 ml of 1.6 M n-butyllithium in hexanes (33.6 mmol) was added over 25 minutes such that the temperature did not exceed -92°C. The mixture was stirred for 1 hour at -90° to -100°C and sulfur dioxide was bubbled gently below the reaction mixture surface until the reaction had ceased. The source of the gas for the subsurface addition was changed briefly to

nitrogen and while maintaining the temperature below -30°C chlorine gas was added until the exothermic reaction stopped. The reaction mixture was warmed to 0-5°C, the excess chlorine gas was removed and 20 ml of 28% ammonia in water was added to the mixture. The mixture was cooled to 2°C and the precipitated solid was collected by filtration. The solids were washed with hexanes and oven-dried to obtain 7.96 g of solid containing 5-pyrimidinesulfonamide as determined by NMR assay.

## Example 3

### p-Acetylbenzenesulfonamide

A 100 ml three-neck round-bottom flask was charged with 10.01 g of p-bromoacetophenone, ethylene ketal (41.2 mmol) and 50 ml of tetrahydrofuran. The solution was cooled to -69°C, and 29 ml of n-butyllithium solution (1.431 molar; 41 mmol) was added to the reaction mixture so that the temperature of the mixture remained below -61°C. The mixture was stirred for one hour at -70°C whereupon 5 ml of liquid sulfur dioxide (110 mmol) was added slowly until no exothermic reaction was apparent. Nitrogen was bubbled below the reaction mixture surface for 30 minutes followed by the addition of chlorine gas for about 5 minutes. A subsurface nitrogen purge was maintained while the reaction mixture was allowed to warm gradually to ambient

temperature. The mixture was chilled to 5°-15°C and was slowly charged with 10 ml of a solution of 28% ammonia in water and 22 ml of acetone so that the temperature remained below 15°C. To the solution was added 41 ml of deionized water and an additional 28% ammonia until a pH of 9 was obtained. The mixture was evaporated under reduced pressure to remove most of the organic solvents. To the residue was added toluene and acetone. The mixture was heated at reflux for 2 hours, cooled to room temperature and filtered. The filtrate was evaporated to provide 5.30 g of solid residue. To 4.28 g of this residue was added 15 ml of methanol, 5 ml of water and 0.33 g of 98% sulfuric acid. The mixture was stirred overnight and cooled. The mixture was filtered and the solids were washed with a cold 50% aqueous acetone solution. The solid was dried in a vacuum oven overnight to obtain 1.35 g of solid containing p-acetylbenzenesulfonamide as determined by HPLC assay.

## Example 4

### n-Butylsulfonamide

A 100 ml three-neck round-bottom flask was charged with 30.0 ml of 1.6 N n-butyllithium (48.0 mmol) and 20 ml of tetrahydrofuran (freshly distilled from sodium/benzophenone). The mixture was cooled to -50°C and sulfur dioxide was bubbled gently below the

surface of the reaction mixture until the exothermic reaction subsided. The temperature was maintained below -30°C. The mixture was purged briefly with nitrogen and then chlorine until all solids dissolved. The solution was warmed to 0°C, the excess chlorine gas was removed by a nitrogen purge, and 8.4 g of 28% ammonia in water was added such that the temperature remained below 20°C. The mixture was stirred overnight and allowed to warm to room temperature with ambient warming. The mixture was cooled to 0-5°C and stirred for 3 hours. The precipitated solids were collected by filtration and washed with hexanes. This material was oven-dried to obtain 7.68 g of solid containing n-butylsulfonamide as shown by NMR.

## CLAIMS

1. A process for preparing a sulfonamide of Formula (1)

$$R^1-SO_2-NH_2 \qquad (1)$$

in which $R^1$ is $C_1-C_{10}$ alkyl, phenyl, substituted phenyl or a 6-membered heteroaromatic ring containing one or two nitrogen atoms and the remainder carbon atoms which comprises:

(a) contacting an organometallic compound of the formula

$$R^1-M$$

in which M is an alkali metal or $M^1X$, in which $M^1$ is Cd, Zn or Mg and X is halogen, with sulfur dioxide to give an organosulfinate metal salt;

(b) treating the organosulfinate metal salt with chlorine or bromine to afford a sulfonyl halide derivative; and

(c) treating the sulfonyl halide with ammonia.

2. A process as claimed in Claim 1 in which M is an alkali metal.

3. A process as claimed in Claim 2 in which the alkali metal is lithium.

4. A process as claimed in Claim 1 in which M is $M^1X$.

5. A process as claimed in Claim 4 in which $M^1X$ is magnesium bromide.

6. A process as claimed in Claim 1 in which $R^1$ is n-butyl, p-acetylphenyl-, 5-pyrimidinyl, or phenyl.

7. A process for preparing a sulfonyl halide of Formula (2)

$$R^1-SO_2-X \qquad (2)$$

in which $R^1$ is $C_1$-$C_{10}$ alkyl, phenyl, substituted phenyl or a 6-membered heteroaromatic ring containing one or two nitrogen atoms and the remainder carbon atoms and X is chlorine or bromine which comprises treating an organosulfinate metal salt of the formula

$$R^1-SO_2-M$$

in which M is an alkali metal or $M^1X$, in which $M^1$ is Cd, Zn or Mg and X is halogen, with chlorine or bromine.

8. A process as claimed in Claim 7 in which M is an alkali metal.

9. A process as claimed in Claim 7 in which M is $M^1X$.

10. A compound of Formula (1) whenever prepared by a process according to Claim 1.

11. A compound of Formula (2) whenever prepared by a process according to claim 7.

European Patent Office

# EUROPEAN SEARCH REPORT

0143627

Application number

EP 84 30 8161

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | JOURNAL OF ORGANOMETALLIC CHEMISTRY, vol. 121, 1976, pages 123-126, Elsevier Sequoia S.A., Lausanne, CH; G. CAHIEZ et al.: "Insertion de l'anhydride sulfureux dans la liaison carbone-cuivre" * Pages 124,126 * | 1,7 | C 07 C 143/72<br>C 07 C 143/70<br>C 07 C 145/00<br>C 07 D 239/38 |
| | --- | | |
| Y | THE JOURNAL OF ORGANIC CHEMISTRY, vol. 45, no. 3, 1st February 1980, pages 406-410, The American Chemical Society; L.K. LIU et al.: "Copper-catalyzed additions of sulfonyl iodides to simple and cyclic alkenes" * Page 409 * | 1,7 | |
| | --- | | TECHNICAL FIELDS SEARCHED (Int. Cl.4) |
| Y | US-A-3 202 707 (C.M. STARKS) * Claims * | 1,7 | C 07 C 143/00<br>C 07 D 239/00<br>C 07 C 145/00 |
| | --- | | |
| Y | FR-A- 836 554 (SOCIETE POUR L'INDUSTRIE CHIMIQUE A BALE) * Page 1, lines 56-62 * | 1,7 | |
| | --- | | |
| Y | CH-A- 387 020 (M & T CHEMICALS INC.) * Claims; examples * | 1,7 | |
| | --- | | |
| Y | FR-A-2 374 287 (UGINE KUHLMANN) * Claims; examples * | 1,7 | |
| | --- -/- | | |

The present search report has been drawn up for all claims

| Place of search | Date of completion of the search | Examiner |
|---|---|---|
| THE HAGUE | 06-03-1985 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

0143627

Application number

EP 84 30 8161



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-2 821 193 (CHEMISCHE FABRIK STOCKHAUSEN)<br>* Claims *<br>----- | 1 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 06-03-1985 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82